# EUROPEAN PATENT SPECIFICATION

(11) **EP 4 507 589 B1**
(45) Date of publication and mention of the grant of the patent: **02.12.2026**
(21) Application number: 23719466.7
(22) Date of filing: 29.03.2023
(51) Int. Cl.: A61B 17/12

(54) **MEDICAL DEVICE DELIVERY MEMBER WITH POSITIONING WINDOW**
MEDIZINISCHES GERÄT LIEFERTEIL MIT POSITIONIERUNGSFENSTER
DISPOSITIF DE LIVRAISON DE DISPOSITIFS MÉDICAUX AVEC FENÊTRE DE POSITIONNEMENT

(30) Priority: 15.04.2022 US 202217721474
(43) Date of publication of application: 19.02.2025
(73) Proprietor: DePuy Synthes Products, Inc., Raynham, MA 02767 (US)
(72) Inventor: GALLERANI, Thomas, Raynham, Massachusetts 02767 (US); SOLAUN, Daniel, Raynham, Massachusetts 02767 (US)
(74) Representative: Carpmaels & Ransford LLP
(86) International application number: PCT/IB2023/053100
(87) International publication number: WO 2023/199147

(56) References cited:
- US-A1- 2014 257 363
- US-A1- 2019 350 589
- US-A1- 2021 219 981
- US-B2- 9 554 805

## Description

### FIELD OF INVENTION

This invention generally relates to intravascular medical device systems that navigable through body vessels of a human subject. More particularly, this invention relates to delivery systems and delivery members for delivering and deploying an implantable medical device to a target location of a body vessel.

### BACKGROUND

The use of catheter delivery systems for positioning and deploying therapeutic devices, such as dilation balloons, stents and embolic coils, in the vasculature of the human body has become a standard procedure for treating endovascular diseases. It has been found that such devices are particularly useful in treating areas where traditional operational procedures are impossible or pose a great risk to the patient, for example in the treatment of aneurysms in cranial blood vessels. Due to the delicate tissue surrounding cranial blood vessels, e.g. brain tissue, it can be difficult and often risky to perform surgical procedures to treat defects of the cranial blood vessels. Advancements in catheter-based implant delivery systems have provided an alternative treatment in such cases. Some of the advantages of catheter delivery systems are that they provide methods for treating blood vessels by an approach that has been found to reduce the risk of trauma to the surrounding tissue, and they also allow for treatment of blood vessels that in the past would have been considered inoperable.

Typically, these procedures involve inserting a delivery catheter into the vasculature of a patient and guiding it through the vasculature to a predetermined delivery site. A vascular occlusion device, such as an embolic coil, can be attached to an implant engagement/deployment system (referred to herein equivalently as an "engagement system" or "deployment system") at a distal end of a delivery member (e.g. micro-catheter) which pushes the coil through the delivery catheter and out of the distal end of the delivery catheter into the delivery site. Example delivery members and engagement/deployment systems are described in U.S. Patent Numbers 10,806,462, 10,806,461, and 11,207,494.

Some of the challenges that have been associated with properly executing such treatment procedures include ensuring the delivery member and engagement system remain in a stable position throughout a treatment. For example, in some aneurysm treatment applications, as the aneurysm becomes increasingly packed with embolic material, the delivery member can tend to shift due to increasing pushback from the embolic material being implanted. If the delivery member shifts during treatment, a physician may not be able to accurately control placement of embolic material and may choose to cease packing the aneurysm. In such an example, the aneurysm may not be sufficiently packed, which can lead to recanalization. Further, excessive movement or stretching of the delivery member and/or engagement system thereon can result in premature detachment of the embolic coil.

There is therefore a need for improved methods, devices, and systems to provide an implant delivery member and implant engagement system with increased stability.
US 2021/0219981 Al describes a delivery member that is provided for delivering and deploying an intravascular medical device. The delivery member includes a flexible distal portion including a wound wire coil surrounded by a flexible sleeve and inhibited from extending lengthwise by a stretch resistant member positioned through the lumen of the coil. The delivery member can include hypotubes positioned on either side ( distally and proximally) from the wound wire coil to which the stretch resistant member and the wound wire coil can be attached. The distal hypotube can include attachment slots for positioning and attaching a loop wire to the distal hypotube.

### SUMMARY

It is an object of the present invention to provide systems, devices, and methods to meet the above-stated needs. Generally, it is an object of the present invention to provide a delivery member for delivering and deploying an implantable medical device having a flexible distal portion. For the purposes of this disclosure, 1 inch is approximately 2.54cm and 1 mils (a thousandth of an inch) is approximately 25.4 µm.

Stiffness of the distal portion of the delivery member can cause the microcatheter used for delivery of the embolic material to pull back out of the aneurysm as the distal end of the delivery member is advanced through the tortuous distal anatomy. If the microcatheter pulls back while advancing the embolic material, the microcatheter may come out of the aneurysm and the physician may lose control of the embolic coil and not be able to accurately control placement of embolic material and may not be able to complete treatment.

Flexibility can be provided by incorporating a length of wound coil along the distal portion of the delivery member. The wound coil can be protected by a flexible polymer sleeve positioned around the outside of the coil. The wound coil can be inhibited from elongating by a stretch resistant tube affixed to hypotubes on either end of the wound coil.

The loop wire can be a metallic material, and the first attachment and/or the second attachment can be a weld.

The first attachment and/or the second attachment can include an epoxy.

The first attachment can be a first knot in the loop wire having a knot diameter approximately equal to the first diameter of the first attachment slot. The second attachment can be a second knot in the loop wire having a diameter approximately equal to the first diameter of the second attachment slot.

The loop wire can be a polymer material.

The distal hypotube can include a spiral cut along a longitudinal axis passing through the lumen.

The first attachment slot can be at a position on the distal hypotube opposite the second attachment slot along a diameter line passing through the longitudinal axis. The first attachment slot can be positioned proximal to the second attachment slot at a distance equal to one-half of a pitch of the spiral cut.

The first attachment slot can be at a first position equidistant from two adjacent cuts of the spiral cut. The second attachment slot can be at a second position equidistant from two adjacent cuts of the spiral cut.

The first attachment slot and the second attachment slot can be perpendicular to the longitudinal axis.

An example distal hypotube for a delivery member can have a distal end shaped to receive an implantable medical device. The distal hypotube can have a lumen extending through the distal hypotube. The distal hypotube can include a first attachment slot extending from the lumen to an outer surface of the distal hypotube. The distal hypotube can include a second attachment slot extending from the lumen to the outer surface. The distal hypotube can have a loop wire. The loop wire can be attached at a first end to the first attachment slot and attached at a second end to the second attachment slot. The distal hypotube can have a spiral cut in the outer surface. The spiral cut can be cut along a longitudinal axis passing through the lumen.

The first attachment slot can have a distal end and a proximal end. The proximal end of the first attachment slot can have a larger diameter than the distal end of the first attachment slot. The second attachment slot can have a distal end and a proximal end. The proximal end of the second attachment slot can have a larger diameter than the distal end of the second attachment slot. The loop wire can be attached to the first attachment slot via a first attachment. The loop wire can be attached to the second attachment slot via a second attachment.

The loop wire can be a metallic material, and the first attachment and/or the second attachment can be a weld.

The first attachment and/or the second attachment can include an epoxy.

The first attachment can be a first knot in the loop wire having a knot diameter approximately equal to the first diameter of the first attachment slot. The second attachment can be a second knot in the loop wire having a diameter approximately equal to the first diameter of the second attachment slot.

The first attachment slot can be at a position on the distal hypotube opposite the second attachment slot along a diameter line passing through the longitudinal axis. The first attachment slot can be positioned proximal to the second attachment slot at a distance approximately equal to one-half of a pitch of the spiral cut.

An example method for attaching an implantable medical device on a delivery member includes providing the delivery member. The delivery member can include a flexible distal hypotube. The method can include passing a first end of a loop wire through a proximal end of a first attachment slot of the flexible distal hypotube. The method can include pulling the first end of the loop wire from the proximal end of the first attachment slot toward a distal end of the first attachment slot, the distal end having a smaller diameter than the proximal end. The method can include attaching the loop wire to the first attachment slot. The method can include passing a second end of the loop wire through a proximal end of a second attachment slot of the flexible distal hypotube. The method can include pulling the second end of the loop wire from the proximal end of the second attachment slot toward a distal end of the second attachment slot, the distal end having a smaller diameter than the proximal end. The method can include attaching the loop wire to the second attachment slot.

Attaching the loop wire to the first attachment slot can include welding the loop wire within the first attachment slot.

The method can include tying a first knot in the first end of the loop wire prior to pulling the first end of the loop wire from the proximal end of the first attachment slot toward

An example delivery member includes a distal hypotube. The distal hypotube has a distal end shaped to receive an implantable medical device. The distal hypotube has a longitudinal axis from the distal end to a proximal end of the distal hypotube. The distal hypotube has a first tube window extending from a lumen of the distal hypotube to an outer surface of the distal hypotube. The first tube window has a length greater than a width, wherein the length is along the longitudinal axis. The delivery member has a support tube positioned within the lumen of the distal hypotube. The support tube is advanceable through the lumen of the distal hypotube such that a distal end of the support tube can be positioned within the length of, and is viewable through, the first tube window.

A distal end of the support tube can be positioned such that it is placed within the length of the first tube window and is visible external to the distal hypotube.

The delivery member can have a loop wire extended through an opening in the implantable medical device. The delivery member can have a pull wire extended through the lumen, engaged to the loop wire, and movable to retract proximally to disengage the loop wire to deploy the implantable medical device.

The distal hypotube can include a first attachment slot extending from a lumen of the distal hypotube to an outer surface of the distal hypotube. A first end of a loop wire can extend at least partially through the first attachment slot.

The distal hypotube can include a second attachment slot extending from the lumen to the outer surface. A second end of the loop wire can extend at least partially through the second attachment slot. The first tube window can be positioned proximal to the first attachment slot and the second attachment slot in relation to the distal end of the distal hypotube.

The length of the first tube window can be from approximately 0.10 inches to 0.40 inches, and the width of the first tube window can be from approximately 0.02 inches to 0.10 inches.

The length of the first tube window can be from approximately 0.2 inches to 0.30 inches, and the width of the first tube window can be from approximately 0.035 inches to 0.86 inches.

The distal hypotube can include a spiral cut along the longitudinal axis. The first tube window can be positioned between adjacent cuts of the spiral cut.

The distal hypotube can include a second tube window. The first tube window and the second tube window can be positioned equidistant from the distal end of the distal hypotube.

An example method for manufacturing a delivery member for an implantable medical device includes passing a support tube through a lumen of a flexible distal hypotube of the delivery member until a distal end of the support tube is positioned within a length of and viewable through a tube window of the flexible distal hypotube. The method includes affixing the support tube to the delivery member. The method includes affixing a loop wire to the flexible distal hypotube. The tube window has a length greater than a width, wherein the length is along a longitudinal axis passing through the lumen.

Affixing the loop wire to the flexible distal hypotube can include passing a first end of a loop wire through a proximal end of a first attachment slot of the flexible distal hypotube. Affixing the loop wire can further include pulling the first end of the loop wire from the proximal end of the first attachment slot toward a distal end of the first attachment slot, the distal end having a smaller diameter than the proximal end. Affixing the loop wire can further include attaching the loop wire to the first attachment slot. Affixing the loop wire can further include passing a second end of the loop wire through a proximal end of a second attachment slot of the flexible distal hypotube. Affixing the loop wire can further include pulling the second end of the loop wire from the proximal end of the second attachment slot toward a distal end of the second attachment slot, the distal end having a smaller diameter than the proximal end. Affixing the loop wire can further include attaching the loop wire to the second attachment slot.

The tube window can be positioned proximal to the first attachment slot and the second attachment slot in relation to the distal end of the flexible distal hypotube.

The method can include forming the tube window in the flexible distal hypotube by cutting or etching the tube window between adjacent cuts of a spiral cut in the flexible distal hypotube.

The length of the tube window can be from approximately 0.10 inches to 0.40 inches, and the width of the tube window can be from approximately 0.02 inches to 0.10 inches.

The length of the tube window can be from approximately 0.2 inches to 0.30 inches, the width of the tube window can be from approximately 0.035 inches to 0.86 inches.

A distal end of the tube window can be positioned approximately 22 mm from the distal end of the flexible distal hypotube.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of a cross section of a delivery member, according to aspects of the present invention;
FIG. 2A is an illustration of a cross section of a flexible sleeve, according to aspects of the present invention;
FIG. 2B is an illustration of a cross section of a stretch resistant tube, according to aspects of the present invention;
FIG. 2C is an illustration of a cross section of a wire coil affixed to a distal hypotube and a proximal hypotube, according to aspects of the present invention;
FIG. 2D through 2H are illustrations of a distal hypotube with first and second attachment slots, according to aspects of the present invention;
FIGs. 3A through 3D are illustrations of an engagement system illustrating a sequence for deploying an implant, according to aspects of the present invention;
FIG. 4 is a flow diagram illustrating a method for designing or constructing a delivery member, according to aspects of the present invention;
FIG. 5 is a flow diagram illustrating a method for using a delivery system including an example delivery member, according to aspects of the present invention;
FIG. 6 is a flow diagram illustrating a method for attaching an implantable medical device to a delivery member, according to aspects of the present invention;
FIG. 7A is an illustration of a delivery member with a tube window and a support tube partially positioned within an inner lumen of the distal hypotube, according to aspects of the present invention;
FIG. 7B is an illustration of a delivery member with a tube window and a support tube fully positioned within an inner lumen of the distal hypotube, according to aspects of the present invention;
FIG. 7C is an illustration of a delivery member, providing a detailed view of a tube window, according to aspects of the present invention;
FIG. 7D is an illustration of an engagement system having a tube window, according to aspects of the present invention; and
FIG. 8 is a flow diagram illustrating a method for manufacturing a delivery member for an implantable medical device, according to aspects of the present invention.

### DETAILED DESCRIPTION

During an intravascular treatment, for instance, an aneurysm occlusion treatment, lack of flexibility of a distal portion of a treatment device delivery member can cause the delivery member to pull back from the treatment site or otherwise move out of position while an implant or other medical treatment device is being placed in an aneurysm or other treatment site. A delivery member and engagement system having a more flexible distal portion can therefore provide a stable system for delivering medical devices in neurovascular anatomy in addition to other applications facing a similar challenge. Flexible structures, however, can tend deform, extend, or expand when navigating tortuous anatomy. Deformation of the delivery member can inhibit the delivery member's ability to navigate to a treatment site and/or effectively deploy the medical device. Elongation of the delivery member can result in premature deployment of the medical device.

An object of the present invention is to provide a delivery member having a highly flexible distal portion that is stretch resistant and structurally stable throughout delivery and deployment of a medical treatment device. For ease of discussion, medical treatment devices are generally referred to herein as an "implant" although, as will be appreciated and understood by a person of ordinary skill in the art, aspects of the present invention can be applied to deliver and deploy medical treatment devices that are not left implanted.

According to the present invention, in some examples, the highly flexible distal portion of the delivery member can include a coiled wire, an outer sleeve, and an inner stretch resistant support tube. The coiled wire can be formed of a substantially linear wire that is wound in a coil shape and/or a hypotube that is laser cut in a spiral pattern. If the coiled wire is formed from a laser cut hypotube, the spiral can be absent interference cuts connecting windings in the coil so as to provide a more flexible coil. The outer sleeve can inhibit the coiled wire from deforming radially and/or provide a smooth surface against which vascular walls can slide during delivery of an implant. The stretch resistant support tube can inhibit elongation of the coiled wire during delivery of the implant. The combination of the coiled wire, outer sleeve, and stretch resistant support tube can therefore provide a distal portion of a delivery member having greater flexibility and greater stability than at least some known delivery members.

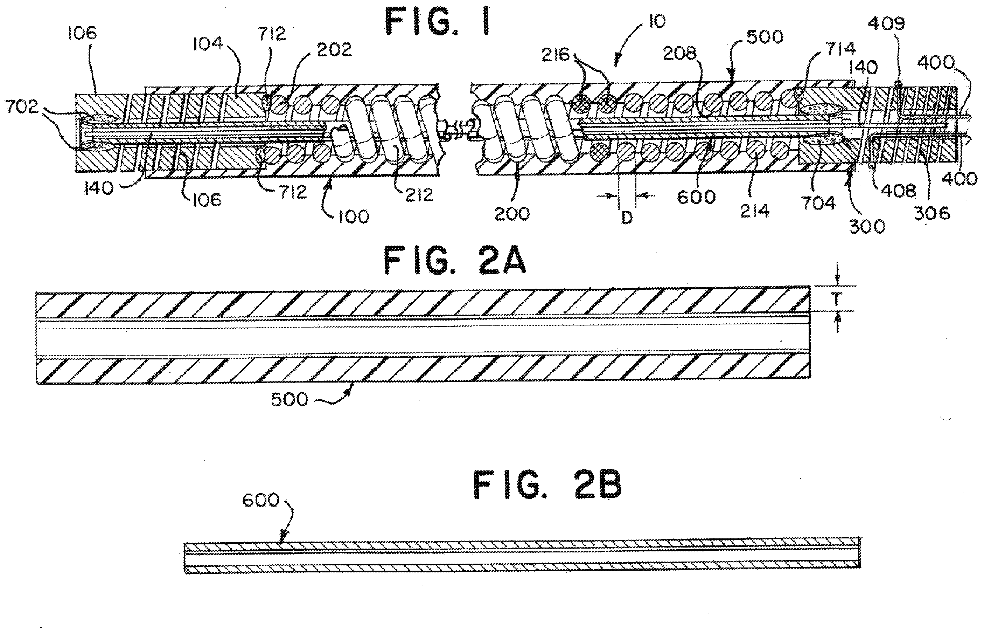

Turning to the figures, as illustrated in FIG. 1, an example delivery member 10 can include a proximal tube 100, a coiled section 200, a distal tube 300, a sleeve 500 surrounding the coiled section, and a stretch resistant support tube 600 within the lumen of the coiled section 200. In this disclosure, the terms proximal tube and proximal hypotube can be used interchangeably; similarly, the terms distal tube and distal hypotube can be used interchangeably. The proximal tube 100 can extend a majority of the length of the delivery member 10 with the coiled section 200 and distal tube 300 forming a length sufficient to absorb a majority of push-back that can occur during placement of an implant at a treatment site. In some examples, the length can measure between about 30 cm and about 50 cm, or more specifically, about 40 cm. The proximal tube 100 can have a distal end 104 that is connected to a proximal end 202 of the coiled section 200, and the coiled section 200 can have a distal end 204 that is connected to a proximal end 302 of the distal tube 300. As will be described in greater detail herein, the distal tube 300 can include a compressible portion 306. The compressible portion 306 can be formed from a spiral cut 307 in the distal tube 300, a wound wire, spiral ribbon, or other arrangement allowing axial adjustment according to the present invention. A loop wire 400, which can secure an implant or other medical treatment device to the delivery member 10, can be placed within the lumen 308 of the distal tube 300. The compressible portion 306 of the distal tube 300 can include attachment slots (e.g., a first attachment slot 310 and a second attachment slot 316) enabling the loop wire 400 to pass through the distal tube 300 from the lumen 308 to an outer surface 309 of the distal hypotube 300.

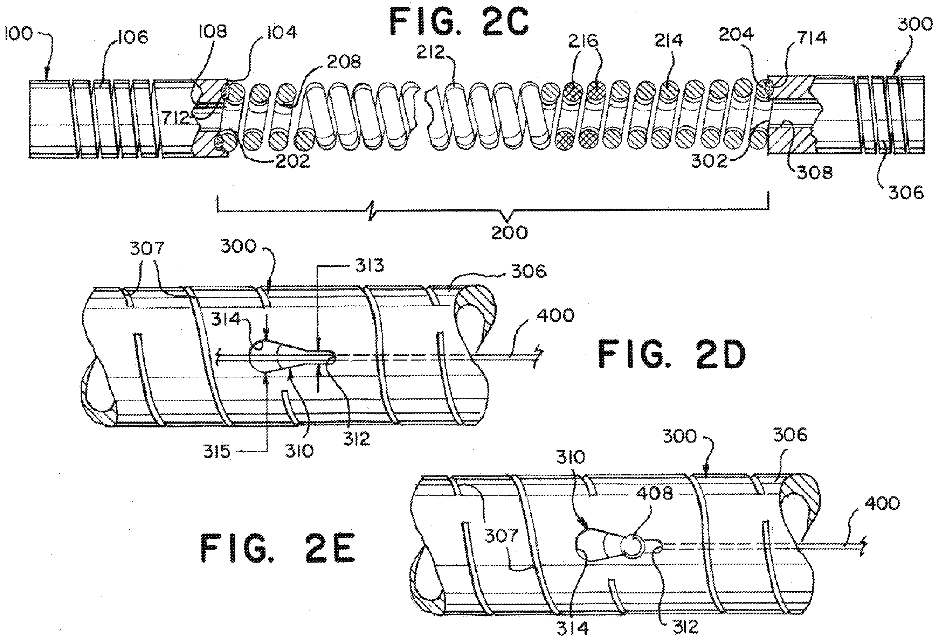

FIG. 2A is a cross sectional view of the sleeve 500. FIG. 2B is a cross sectional view of the stretch resistant support tube 600. FIG. 2C is a cross sectional view of the assembled proximal tube 100, coiled section 200, and distal tube 300.

The coiled section 200 can be formed separately from the proximal hypotube 100 and/or the distal hypotube 300. The separately formed coiled section 200 can be affixed with welds 712, 714 or other appropriate attachment to the proximal tube 100 and/or the distal tube 300. Alternatively, or additionally, at least a portion of the coiled section can be formed from a spiral laser cut portion of a hypotube. A separately formed coiled section 200 can be made more flexible compared to a spiral cut tube by selecting a wire with a particular cross section (e.g. circular) with a particular diameter D, or by selecting a wire with material properties to increase flexibility. Conversely, a laser cut portion can be more easily fabricated by cutting a single hypotube to form the proximal tube 100, coiled section 200, and distal hypotube 300, reducing or eliminating welds 712, 714 or other attachments. In either case, the wire of the coil 200 can have a diameter D measuring within a range including about 0.8 mils and 5 mils (about 20 nm to about 130 nm).

The coiled section can be formed primarily of a non-radiopaque material such as steel and can include a radiopaque section 216 made of a radiopaque material such as platinum and/or tungsten. The radiopaque section 216 can be positioned between a proximal, non-radiopaque section of the coil 212 and a distal, non-radiopaque section of the coil 214. The radiopaque section 216 can be positioned a predetermined distance from a distal end 304 of the delivery member 10 so that a physician can readily visualize the placement of the distal portion of the delivery member during a treatment procedure. The proximal section 212, radiopaque section 216, and distal section 214 can be concentrically welded.

The coiled section 200 can be surrounded by a flexible sleeve or fused sleeve 500, referred generically herein as a "sleeve." The sleeve 500 can inhibit the coil 200 from expanding radially and/or from engaging vascular walls during navigation. The sleeve 500 can include a polymer. The polymer can include additives to increase the lubricity of the sleeve 500 so that the sleeve can easily slide through a body vessel. As illustrated in FIG. 2A, the sleeve 500 can have a wall thickness T measuring within a range including about 0.5 mils and about 2 mils (about 0.01 mm to about 0.05 mm). The sleeve 500 can further be coated with a hydrophilic coating to further minimize friction during intravascular navigation. The sleeve 500 can be fused or glued to the coil 200, the proximal hypotube 100, and/or the distal hypotube 300.

The stretch resistant support tube 600 can be positioned to inhibit elongation of the coil 200 during intravascular navigation. The stretch resistant support tube 600 can include a tube sized to fit within the lumen 208 of the coil 200. The stretch resistant tube 600 can also be sized to extend through the entirety of the length of the coil 200, extend with a lumen 108 of the proximal tube 100 and within the lumen 308 of the distal tube 300. The stretch resistant support tube 600 can be attached to the proximal tube 100 and the distal tube 300 at adhesive joints 702, 704 or other appropriate attachment. The stretch resistant support tube 600 can remain unattached to the coiled section 200 such that the stretch resistant support tube 600 and coiled section 200 are able to move independently from each other to some extent.

The delivery member 10 can include a mechanical engagement system for engaging a medical device 12 during delivery to a treatment site, and the delivery member 10 can be actuated mechanically to deploy the medical device 12. Mechanically actuated engagement systems can include one or more inner elongated members or pull wires extending through the delivery member that can be manipulated at the proximal end by a physician to deploy a medical treatment device. Such a wire or inner elongated member is referred to herein generically as a "pull wire." When reference is made herein to an engagement system, the engagement system can include the combination of a loop wire 400 and a pull wire 140.

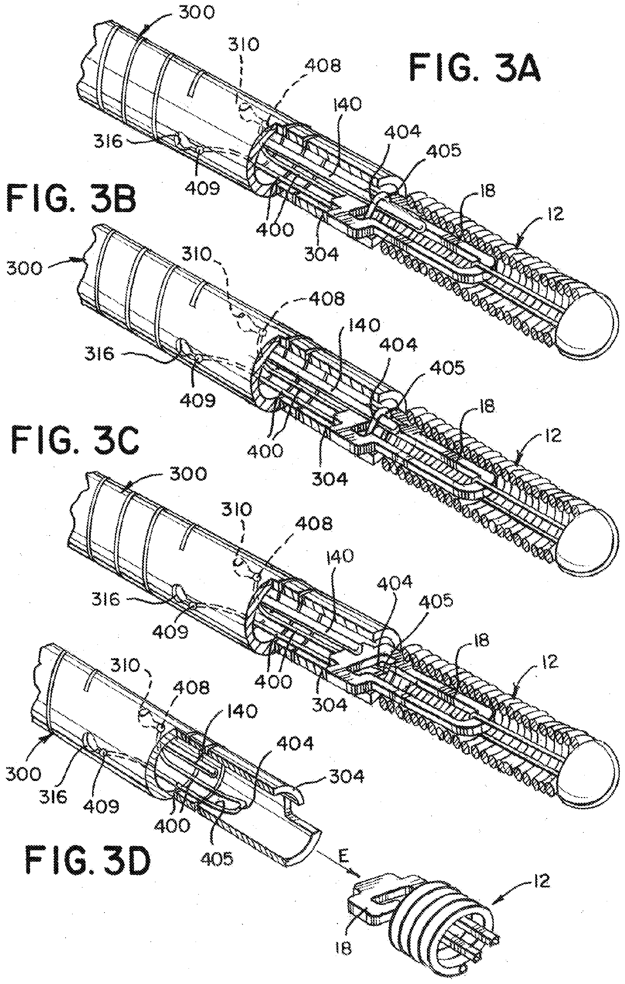

FIGs. 2D through 2H illustrate an example distal hypotube 300 that can be used to secure a loop wire 400. The loop wire 400 can be positioned to secure an implant or other medical treatment device to the delivery member 10 and can be moved to release the medical device 12 from the implant or other medical treatment device. This convention will be described in greater detail below in reference to FIGs. 3A-3D. To summarize, the loop wire 400 can extend over a locking portion 18 of a medical device 12. A pull wire 140 can then be slid through an opening 405 at the distal end 404 of the loop wire 400. This can hold the medical device 12 in place until the pull wire 140 is retracted to release the medical device 12.

FIG. 2D is a side view of a distal hypotube 300 with a first attachment slot 310 and a second attachment slot 316 (the second attachment slot 316 is in the background of the figure). The attachment slots 310,316 can be openings in the outer surface 309 of the distal hypotube 300 that extend from the lumen 308 to the outer surface 309. The attachment slots 310,316 can facilitate attaching the loop wire 400 to the distal hypotube 300. The attachment slots 310,316 can be laser cut, for example, into the outer surface 309 of the distal hypotube 300.

The first attachment slot 310 can have a distal end 312 and a proximal end 314. As can be seen, a diameter 313 of the distal end 312 can be smaller than a diameter 315 of the proximal end 314 of the first attachment slot 310. This tapered (or teardrop) design can facilitate the attachment of the loop wire 400 to the distal hypotube 300. For example, if the proximal end 314 is larger in diameter, the loop wire 400 can be fed through more easily. Once through the larger proximal diameter 315, the loop wire 400 can be pulled toward the distal end (312) of the first attachment slot (310). In some examples, the transition between the proximal end 314 and the distal end 312 can be slightly tapered such that, as the loop wire 400 is pulled toward the distal end 312, the loop wire 400 can be wedged within the first attachment slot. The second attachment slot 316 can be similar in all regards to the first attachment slot 310. For example, the second attachment slot 316 can have a distal end 322 and a proximal end 324, and a diameter 323 of the distal end 322 can be smaller than a diameter 325 of the proximal end 324 of the second attachment slot 316.

When the loop wire 400 is fed through the attachment slot 310,316, and pulled toward the distal end 312,322, the loop wire 400 can be attached to the distal hypotube 300 via an attachment 408,409. FIG. 2E depicts an example first attachment 408 (the second attachment 409 is in the background of this figure). Using the first attachment slot 310 as an example (the second attachment slot 316 can be similar), the loop wire 400 can be pulled toward the distal end 312 and positioned within the first attachment slot 310 at a desired location within the length of the first attachment slot 310. The loop wire 400 can then be attached to the first attachment slot 310 at a first attachment 408. The first attachment 408 and/or the second attachment 409 can be disposed at the distal end of their respective attachment slot 310,316. It is not necessary, however, that the first attachment 408 and/or the second attachment 409 be disposed at the most distal end of the slots; the first attachment 408 and/or the second attachment 409 can be placed at a middle section of the slot (i.e., more proximal), as shown in FIG. 2E.

The first attachment 408 can be a weld that holds the loop wire 400 to the first attachment slot 310. In some examples, the loop wire 400 can comprise metallic materials, such as stainless steel, cobalt-chrome alloy, titanium, nickel-titanium alloy (nitinol), and the like. Similarly, the distal hypotube can comprise metallic materials, such as stainless steel, cobalt-chrome alloy, titanium, nitinol, and the like. This can enable the first attachment 408 to be a metallic weld attaching the loop wire 400 to the first attachment slot 310. In some examples, the loop wire 400 can comprise a polymer material, such as nylon, polypropylene, silk, polyester, and the like. The loop wire can include braided or monofilament wires. The first attachment 408 can be a thermoplastic weld at the first attachment slot 310.

Additionally, or alternatively, the first attachment 408 can be an epoxy holding the end of the loop wire 400 to the first attachment slot 408. As stated above, the second attachment slot 316 can include a second attachment 409 to attach the loop wire 400 to the second attachment slot 316. The second attachment 409 can be similar to the first attachment 408.

In some examples, the first attachment 408 can be a knot in the loop wire 400. As described above, the first attachment slot 310 can have a proximal end 314 that is larger in diameter than the distal end 312. The loop wire 400 can be pulled through the first attachment slot 310, tied into a knot, and then pulled toward the distal end 312. The knot can be larger than the distal diameter 313 so that the loop wire 400 cannot be pulled back through the first attachment slot 310 once a knot is tied in the loop wire 400. In other examples, the loop wire 400 can be tied into a knot first, then the knot can be fed through the proximal end of 314 of the first attachment slot 310, and then the loop wire 400 can be pulled toward the distal end 312. In these examples, a knot diameter can be approximately equal to the first diameter 315 of the first attachment slot 310. This would enable the loop wire 400 to be fed through the proximal end 314 but prohibit the loop wire 400 from being pulled through the distal end 312 of the first attachment slot 310. The second attachment 409 can similarly be a knot in the opposite end of the loop wire 400.

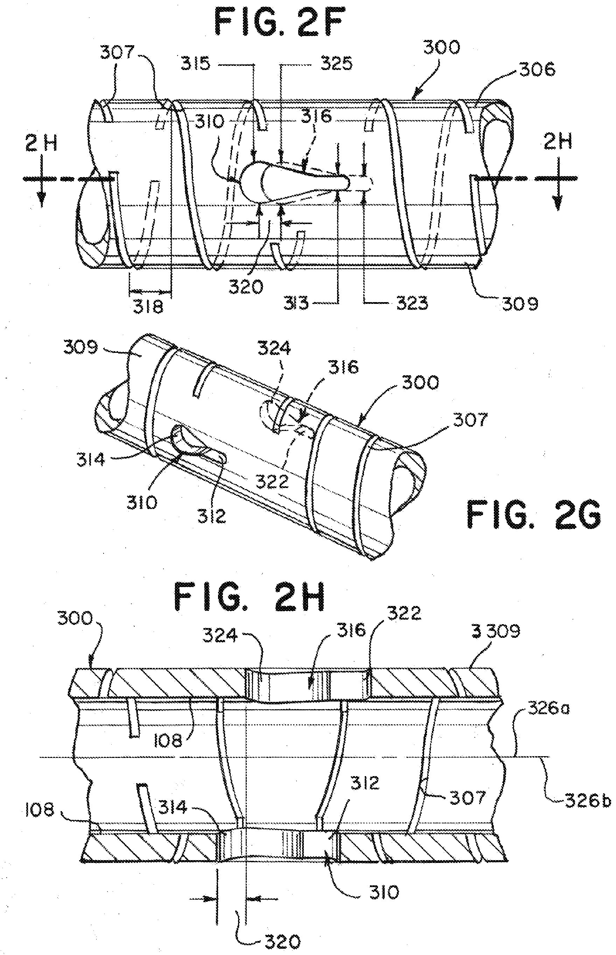

FIG. 2F is a side view of an example distal hypotube 300. The first attachment slot 310 is in the foreground of this figure; the second attachment slot 316 is in the background. As described above, a distal hypotube 300 can have a flexible and/or compressible portion 306. The compressible portion 306 can be formed from a spiral cut 307 in the distal hypotube 300, a wound wire, spiral ribbon, or other arrangement allowing axial adjustment according to the present invention. In some examples, the placement of the first attachment slot 310 and/or the second attachment slot 316 can be selected based on a pitch of the spiral cut 307. To improve the flexibility of the distal hypotube 300, for example, the first attachment slot 310 can be offset from the second attachment slot 316 by a ratio of the pitch of the spiral cut 307, as shown by slot offset 320. As shown in the figure, an example of this slot offset 320 is that the first attachment slot 310 can be positioned opposite the second attachment slot 316, and the first attachment slot 310 can be offset proximal to the second attachment slot 316 at a distance approximately equal to half of the pitch 318 of the spiral cut 307. It is not necessary, however, that the first attachment slot 310 be positioned opposite the second attachment slot 316. The first attachment slot 310 can be disposed at a first position equidistant from two adjacent cuts of the spiral cut 307, and the second attachment slot 316 can disposed at a second position equidistant from two adjacent cuts of the spiral cut 307. This can be true for any position on the distal hypotube 300, whether or not the attachment slots 310,316 are opposite from each other on the outer surface 309.

FIG. 2G is a perspective view of a distal hypotube 300 showing the example offsetting of the first attachment slot 310 and second attachment slot 316. Offsetting the first attachment slot 310 and the second attachment slot 316 with respect to the pitch of the spiral cut 307 can increase the flexibility of the distal hypotube 300 by not impeding its flexing about the spiral cut 307.

FIG. 2H is a side cross-sectional view of an example distal hypotube 300. In some examples, the first attachment slot 310 and the second attachment slot 316 can be formed (for example laser cut, drilled, etc.) into the distal hypotube 300 perpendicular to a longitudinal plane 326b passing through the lumen 308 of the distal hypotube 300. The longitudinal plane 326b is the three-dimensional view of the longitudinal axis 326b passing through the lumen 308. Perpendicular cuts can help facilitate the attachment of the loop wire 400 to the first attachment slot 310 and the second attachment slot 316. For example, once the loop wire 400 is fed through the first attachment slot 310 and the second attachment slot 316, perpendicular cuts can help ensure uniform surface contact between the loop wire 400 and the outer walls of the attachment slots 310,316.

FIG. 2H also provides a good view of an example slot offset 320 between the first attachment slot 310 and the second attachment slot 316. As described above, a first attachment slot 310 can be disposed at a position on the distal hypotube 300 opposite the second attachment slot 316 along a diameter line passing through the longitudinal axis 326a. The first attachment slot 310 and the second attachment slot 316 can be offset based on the pitch of the spiral cut 307.

FIGs. 3A through 3D illustrate the delivery member 10 including a mechanical engagement system including a pull wire 140 and a loop wire 400 that can be positioned to secure an implant or other medical treatment device to the delivery member 10 and can be moved to release the medical treatment device from the delivery member 10. The loop wire 400 can be affixed to the distal tube 300 with first attachment 408 and a second attachment 409. The stretch resistant support tube 600 can be sized to allow a pull wire 140 to pass through the lumens 108, 208, 308 of the proximal tube 100, coiled section 200, and distal tube 300. For instance, the stretch resistant member 600 can be tubular, having a lumen therethrough, and the pull wire 140 can extend through the lumen of the tubular stretch resistant support tube 600. During manufacture of the stretch resistant support tube 600, the stretch resistant support tube 600 can be extruded over the pull wire 140.

The combination of the coil 200, sleeve 500, and stretch resistant support tube 600 can provide a highly flexible distal portion of a delivery member 10 suitable for navigating tortuous anatomy, including neurovascular blood vessels. The stretch resistant support tube 600 can support the coil 200 to prevent the coil 200 from significantly extending during navigation of a blood vessel, thereby reducing tension on a pull wire 140 extending therethrough and reducing the likelihood of premature deployment of an attached medical treatment device.

The proximal tube 100 can include a flexible section 106 having material removed to increase flexibility of the flexible section 106. The flexible section 106 can be cut in a spiral pattern. The spiral pattern of the flexible section 106 can lack interference cuts connecting windings within the spiral. The stretch resistant support tube 600 can extend through the flexible section 106 and be attached to the proximal tube 100 in the proximal direction from the flexible section 106. The stretch resistant support tube 600 can thereby inhibit elongation of the flexible section 106 of the proximal tube 100 and coiled section 200. The sleeve 500 can cover at least a portion of the flexible section 106 to inhibit deformation of the flexible section and/or reduce friction with vasculature and the flexible section 106 during intravascular navigation. In some examples, the sleeve 500 can cover about 10 cm of the proximal tube 100 approximate and/or including the distal end 104 of the proximal tube 100.

The distal hypotube 300 can include a compressible portion 306. The compressible portion 306 can be axially adjustable between an elongated condition and a compressed condition. The distal hypotube 300 can also be flexible such that the distal hypotube 300 can provide a stable system for delivering medical devices in neurovascular anatomy. The distal hypotube 300 can include a spiral cut 307, formed by a laser cutting operation for example, to create the compressible and/or flexible construct. Additionally, or alternatively, the compressible portion 306 can be formed of a wound wire, spiral ribbon, or other arrangement allowing axial adjustment according to the present invention. Preferably, the compressible portion 306 is in the elongated condition at rest and automatically or resiliently returns to the elongated condition from a compressed condition, unless otherwise constrained.

FIGs. 3A-3D, illustrate the detachment of the medical device 12 using a mechanical engagement/deployment system. FIG. 3A illustrates the engagement system 140, 400 locked into the locking portion 18 of the medical device 12. The compressible portion 306 of the distal tube 300 can be compressed and the loop wire 400 opening 405 at a distal end 404 of the loop wire 400 can be placed through the locking portion 18. When the pull wire 140 is put through the opening 405 the medical device 12 is now secure. FIG. 3B illustrates the pull wire 140 being drawn proximally to begin the release sequence for the medical device 12. FIG. 3C illustrates the instant the pull wire 140 exits the opening 405 and is pulled free of the loop wire 400. The distal end 404 of the loop wire 400 falls away and exits the locking portion 18. As can be seen, there is now nothing holding the medical device 12 to the delivery member 10 (e.g., the distal hypotube 300). FIG. 3D illustrates the end of the release sequence. Here, the compressible portion 306 has extended/returned to its original shape and "sprung" forward. An elastic force E is imparted by the distal end 304 of the distal tube 300 to the medical device 12 to "push" it away to ensure a clean separation and delivery of the medical device 12.

Illustrations in the above-described figures depict generally hollow or tubular structures 100, 200, 300, 500, 600 according to the present invention. When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

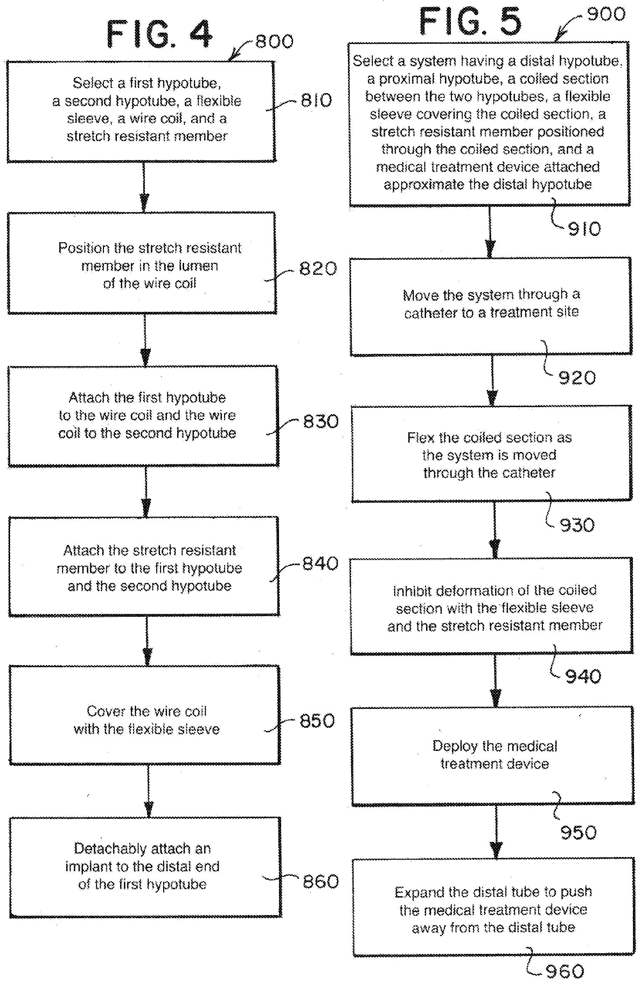

FIG. 4 is a flow diagram including method steps for constructing or designing a delivery member such as the example delivery members described herein. Referring to the method 800 outlined in FIG. 4, in step 810, a first hypotube, a second hypotube, a flexible sleeve, a wire coil, and a stretch resistant support tube can be selected. The first hypotube can be a proximal hypotube 100 as described herein or as would otherwise be known to a person of ordinary skill in the art. The second hypotube can be a distal hypotube 300 as described herein or as would otherwise be known to a person of ordinary skill in the art. The flexible sleeve can be a sleeve or fused jacket 500 as described herein or as otherwise known to a person of ordinary skill in the art. The wire coil can include the support coil, coiled section 200 as described herein or as otherwise known to a person of ordinary skill in the art. The stretch resistant member can be a stretch resistant support tube 600 as described herein or as otherwise known to a person of ordinary skill in the art.

In step 820, the stretch resistant member can be positioned in the lumen of the wire coil. In step 820, the stretch resistant member that is positioned can be substantially tubular. In step 830, the first hypotube, wire coil, and second hypotube can be attached to each other. In step 840, the stretch resistant member is attached to the first hypotube and the second hypotube. The first hypotube, wire coil, and second hypotube can be attached as illustrated and described herein or by other means as would be understood by a person of ordinary skill in the art. Steps 820, 830, and 840 need not be performed in that order and can be performed simultaneously. For instance, the stretch resistant member can be attached to one of the first and second hypotubes as indicated in step 840, then the hypotube to which the stretch resistant member is attached can be attached to the wire coil as indicated in step 830, then the stretch resistant member can be positioned through the wire coil as indicated in step 820, then the other of the hypotubes can be attached to the wire coil as indicated in step 830, then the stretch resistant member can be attached to that other hypotube as indicated in step 840.

In step 850, the wire coil can be covered with the flexible sleeve. The flexible sleeve can cover some or all of the outer surface of the wire coil. Step 850 can also include the step of fusing the flexible sleeve to the wire coil and/or otherwise affixing the flexible sleeve to the delivery member. If the second hypotube has a flexible section, in step 850, the flexible sleeve can also be positioned to cover at least a portion of the flexible section.

In step 860, an implant can be detachably attached to the distal end of the first hypotube. In step 860, the implant can be attached by positioning a loop wire within the first hypotube, positioning a pull wire to extend through the first hypotube, coiled wire, and second hypotube, and securing the implant with the loop wire and the pull wire. The pull wire can be extended from the proximal end of the second hypotube. If the first hypotube has a compressible portion, in step 860, the compressible portion can be compressed, and the implant can be attached to delivery member while the compressible portion is compressed.

FIG. 5 is a flow diagram including method steps for administering an intravascular treatment using a system including a delivery member such as the example delivery members described herein. Referring to the method 900 outlined in FIG. 5, in step 910 a system having a distal hypotube, proximal hypotube, coiled section co-axially positioned in between the hypotubes, a flexible sleeve covering the coiled section, a stretch resistant member positioned within the coiled section, and a medical treatment device attached to or near the distal hypotube can be selected. The system can be suitable for intravascular treatments such as described and illustrated herein or as otherwise known to a person of ordinary skill in the art.

In step 920, the system can be moved through a catheter to a treatment site such as the site of an aneurysm or other abnormality in a blood vessel. In step 930, the system can be flexed as it is moved through the catheter. In step 940, the coiled section of the system can be prevented from deforming by the flexible sleeve and the stretch resistant member; the flexible sleeve can inhibit the coiled section from deforming radially while the stretch resistant member can inhibit the coil from extending longitudinally.

In step 950, the medical treatment device can be deployed. In the case that the medical treatment device is an implant, in step 950 the implant can be detached. In step 960, the distal tube can extend to push the medical treatment device away from the distal tube. In the case that the medical treatment device is an implant detached in step 950, in step 960, the detached implant can be ejected away from the distal tube in response to the expansion of the distal tube.

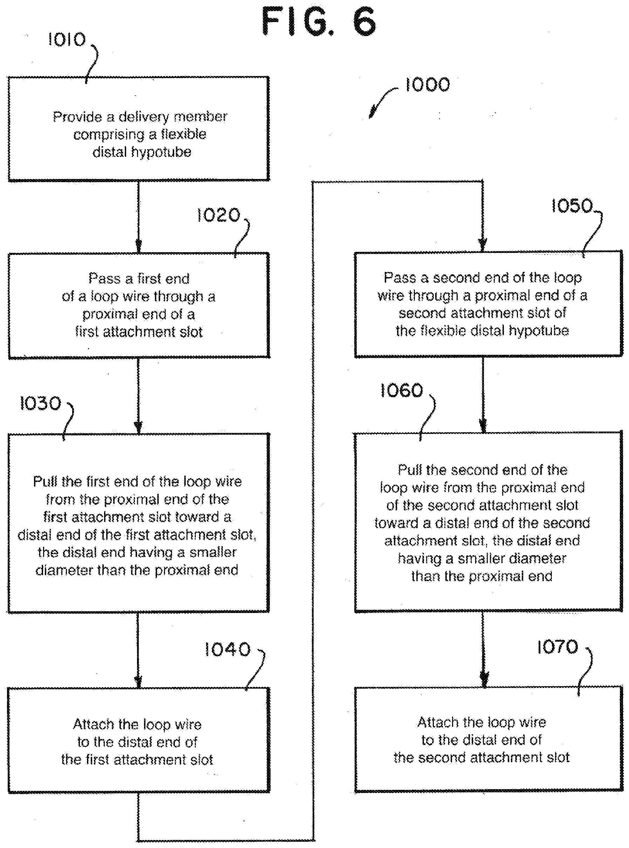

FIG. 6 is a flow diagram including method steps for attaching an implantable medical device to a delivery member. Referring to method 1000 outlined in FIG. 6, in step 1010, the delivery member can be provided. The delivery member can include a flexible distal hypotube, as described herein. In step 1020, a first end of a loop wire can be passed through a proximal end of a first attachment slot of the distal hypotube. In step 1030, the first end of the loop wire can be pulled from the proximal end toward a distal end of the first attachment slot. As stated above, the distal end of the first attachment slot can have a smaller diameter than the proximal end.

In step 1040, the loop wire can be attached to the first attachment slot. This attachment can be made in a number of ways, as described herein. The loop wire can be welded to the first attachment slot. In other examples, the loop wire can be affixed to the first attachment slot via an epoxy. In other examples, the loop wire can include a knot tied into the first end of the loop wire. The knot can be tied prior to pulling the first end of the loop wire through the proximal end of the first attachment slot. In this case, the knot can be approximately the same size as the diameter of the proximal end of the first attachment slot. This can enable the knot to be fed through the proximal end but prohibit the knot from being pulled back through the distal end. The knot can be tied after pulling the first end of the loop wire through the proximal end of the first attachment slot. The knot can be larger than one or both of the proximal and the distal end of the first attachment slot so that the loop wire cannot be pulled back through the first attachment slot. The first end of the loop wire can be attached at the distal end of first attachment slot, or the first end of the loop wire can be attached at any other location in the first attachment slot that is narrower than the proximal end (see, for example, FIG. 2E).

In step 1050 through step 1070, the method steps outlined above for steps 1020 through 1040 can be repeated for a second attachment slot. For example, a second end of the loop wire can be positioned in a second attachment slot similar to the process described above for the first end of the loop wire and the first attachment slot.

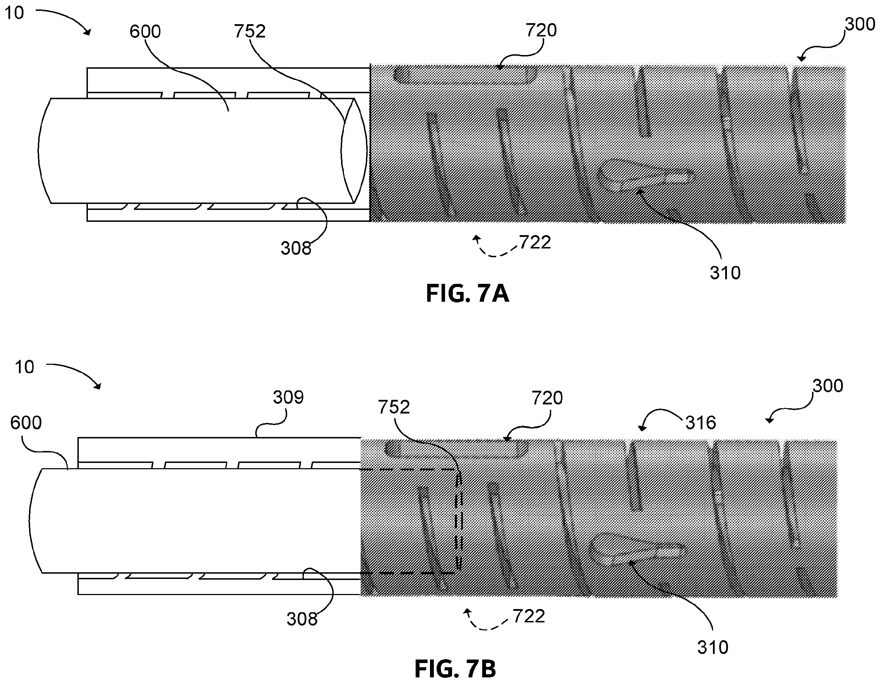

FIGs. 7A through 7D depict a delivery member 10 that includes one or more tube windows 720 positioned at the distal hypotube 300. In some examples, a delivery member 10 can include a support tube (e.g., the stretch resistant support tube 600) to provide support to the attachment of the coiled section 200 and the distal tube 300. Referring briefly to FIG. 2C to illustrate, a distal end 204 of the coiled section 200 abuts a proximal end 302 of the distal tube 300. To provide support to this junction, the delivery member 10 can include a support tube 600 passing across this junction and at least partially into the lumen 308 of the distal tube 300. The support tube 600 can be affixed to the delivery member 10, for example via an adhesive so as to provide support to the junction between the coiled section 200 and the distal tube 300. In some examples, the sleeve 500 described herein (see for example FIG. 1) can be positioned about the outer surface 309 of the distal hypotube 300, and the sleeve 500 can be melted along the distal tube 300 and coiled section 200 over the area where the support tube 600 is affixed. A combination of the affixed support tube 600 (e.g., via adhesives like glues) and the melted sleeve 500 (which can penetrate the coils of the coiled section 200 and adhere to the support tube 600) can assist in holding the components together. The support tube 600 can be manufactured from a polymer material, such as polyether ether ketone (PEEK) and other polymers.

The placement of the support tube 600 can be important-as improper placement can lead to manufacturing difficulties. For example, if the support tube 600 is placed too proximal, proper support at the junction between the distal tube 300 and coiled section 200 may not be achieved; if the support tube 600 is placed too distal, the support tube 600 can interfere with the placement of the attachments (e.g., attachments 408,409) at the attachment slots (e.g., first attachment slot 310 and second attachment slot 316). The present disclosure provides a solution assist in placement of the support tube 600 by using one or more tube windows 720, as shown in FIGs. 7A through 7D.

Referring to FIG. 7A, the delivery member 10 can include a first tube window 720. The first tube window 720 can extend from a lumen 308 of the distal hypotube 300 to an outer surface 309 of the distal hypotube 300. As such, the first tube window 720 can create an aperture through which the support tube 600 can be visualized. The first tube window 720 can have a length (L) greater than a width (W), wherein the length (L) is along the longitudinal axis 326b passing through the lumen 308 of the distal hypotube 300. The longitudinal axis 326b is shown in detail in FIG. 2H.

In some examples, the delivery member 10 can include a second tube window 722, which can be similar to the first tube window 720, such that the position of the support tube 600 within the lumen 308 can be viewed at different angles. In examples with two tube windows, the first tube window 720 and the second tube window 722 can be positioned equidistant from the distal end 304 of the distal hypotube 300. Throughout this disclosure, when reference is made to a singular "tube window," it can be referred to as "tube window 720"; but it will be appreciated that any description of a tube window 720 can apply equally to the first tube window or the second tube window 722. The tube window 720 can be formed into the distal hypotube 300 by cutting or etching the tube window 720 into the distal hypotube 300. In some examples, the tube window 720 can be cut or etched between adjacent cuts of a spiral cut 307 in the distal hypotube 300.

FIG. 7A is an illustration of a delivery member 10 with a tube window 720 and a support tube 600 partially positioned within an inner lumen 308 of the distal hypotube 300. In other words, the support tube 600 has not been inserted to its final position in FIG. 7A. FIG. 7B is an illustration of a delivery member with a tube window and a support tube 600 fully positioned within an inner lumen 308 of the distal hypotube 300, according to aspects of the present invention. The tube window 720 can be positioned at a distance 726 from the distal end 304 of the distal hypotube 300 to ensure that the support tube 600 is properly placed. To illustrate, when manufacturing the delivery member 10 by inserting the support tube 600 into the device, it can be determined that the support tube 600 is inserted to the correct positioned if a distal end 752 of the support tube 600 is visible within the length (L) of the tube window 720. If none of the support tube 600 is visible within the tube window 720, then the support tube 600 is not inserted far enough; if the support tube 600 is visible in the tube window 720 but the distal end 752 of the support tube 600 is not, then the support tube 600 is inserted too far.

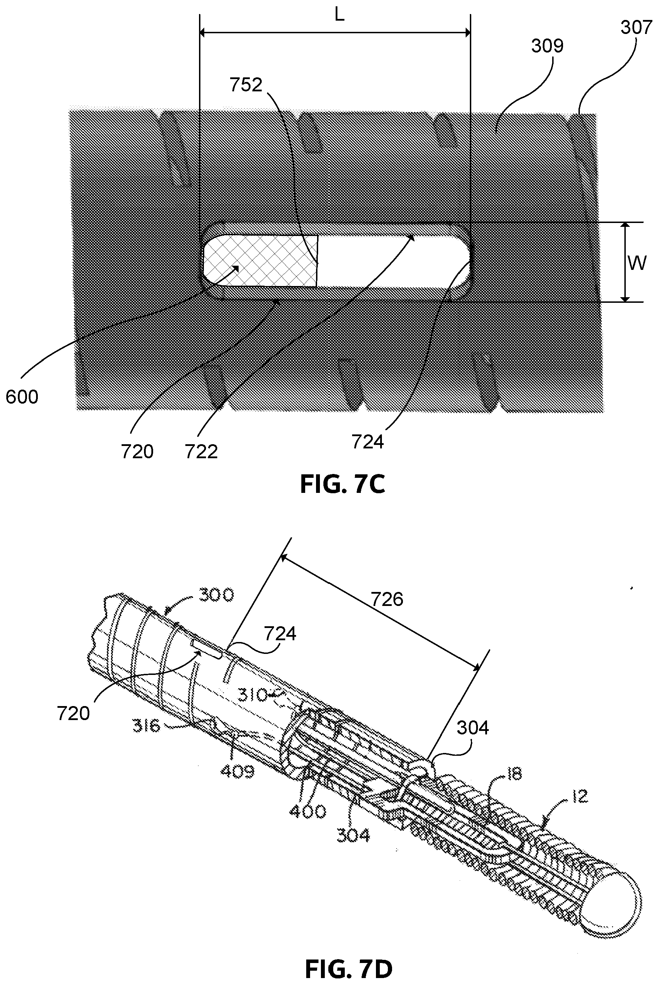

FIG. 7C is a side illustration of a delivery member 10, and the side view provides a detailed view of a tube window 720, according to aspects of the present invention. The view shows a finally-placed support tube 600, as the distal end 752 of the support tube 600 is visible within the length (L) of the tube window 720. The example delivery member 10 shown in FIG. 7C includes a second tube window 722, as indicated by being able to view directly through the first and second windows. The length (L) of the tube window 720 can be from approximately 0.10 inches to approximately 0.40 inches, for example the length (L) of the tube window 720 can be from approximately 0.2 inches to approximately 0.30 inches. The width (W) of the tube window 720 can be from approximately 0.02 inches to approximately 0.10 inches, for example the width (W) of the tube window 720 can be from approximately 0.035 inches to 0.86 approximately inches. These dimensions can provide a view of the support tube 600 within the window(s) while also maintaining the structural integrity of the distal tube 300 and the spiral cut 307.

FIG. 7D is an illustration of an engagement system having a tube window 720, according to aspects of the present invention. The view in FIG. 7D is similar to the view in FIG. 3A, but the delivery member 10 in FIG. 7D includes a tube window 720 in addition to the attachment slots 310,316 described herein. It will be appreciated that any of the features described above with reference to FIGs. 1 through 6 can be applied to a delivery member 10 that includes one or more tube windows 720. Referring again to FIG. 7D, the first tube window 720 (and second tube window 722 if present) can be positioned proximal to the distal end 304 of the distal tube 300 at a position that does not interfere with the attachments 408,409 at the attachment slots 310,316. For example, a distal end 724 of the tube window 710 can be positioned at a distance 726 from approximately 15mm to approximate 25mm from the distal end 304 of the distal hypotube 300, for example approximately 22mm the distal end 304 of the distal hypotube 300.

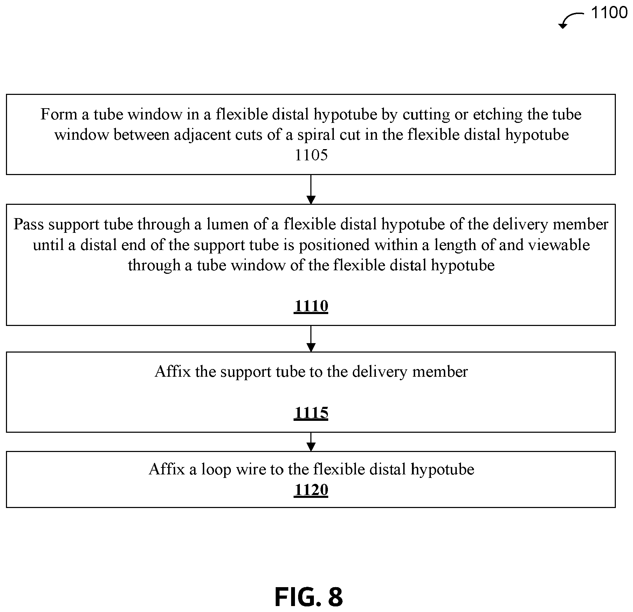

FIG. 8 is a flow diagram illustrating a method 1100 for manufacturing a delivery member 10 for an implantable medical device 12, according to aspects of the present invention. Method 1100 can begin at block 1105, which can include forming a tube aperture a tube window (e.g., first tube window 720 and/or second tube window 722) in a distal hypotube 300 by cutting or etching the tube window 720 between adjacent cuts of a spiral cut 307.

At block 1110, method 1100 can include passing a support tube 600 through a lumen 308 of a flexible distal hypotube 300 of the delivery member 10. The support tube 600 can be advanced until a distal end 752 of the support tube 600 is positioned within a length (L) of and viewable through a tube window 720 of the flexible distal hypotube 300.

At block 1115, method 1100 can include affixing the support tube 600 to the delivery member 10. As described above, the support tube 600 can be affixed to the delivery member 10, for example via an adhesive that is placed between the support tube 600 and (i) the distal hypotube 300 and (ii) a coiled section 200 of the delivery member 10. At block 1120, method 110 can include affixing a loop wire 400 to the flexible distal hypotube 300. In some examples, the method of affixing the loop wire can be performed according to steps 1020 to 1070 described above with reference to FIG. 6 and method 1000.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

The descriptions contained herein are examples of embodiments of the invention and are not intended in any way to limit the scope of the invention. As described herein, the invention contemplates many variations and modifications of the delivery system, delivery member, and engagement system, including alternative configurations of components, alternative materials, alternative medical treatment devices, alternative means for deploying the medical treatment device, alternative geometries of individual components, alternative means for attaching component parts, etc.

## Claims

1. A delivery member (10) comprising:
a distal hypotube (300) comprising:
a distal end (304) shaped to receive an implantable medical device (12);
a longitudinal axis from the distal end to a proximal end of the distal hypotube; and
a first tube window (720) extending from a lumen (308) of the distal hypotube to an outer surface (309) of the distal hypotube, the first tube window having a length greater than a width, wherein the length is along the longitudinal axis; and
a support tube (600) positioned within the lumen of the distal hypotube
**characterised in that** the support tube is advanceable through the lumen of the distal hypotube such that a distal end (752) of the support tube can be positioned within the length of, and is viewable through, the first tube window.

2. The delivery member of claim 1, wherein the distal end of the support tube is positioned such that it is placed within the length of the first tube window and is visible external to the distal hypotube.

3. The delivery member of claim 1, further comprising:
a loop wire (400) extended through an opening in the implantable medical device; and
a pull wire (140) extended through the lumen, engaged to the loop wire, and movable to retract proximally to disengage the loop wire to deploy the implantable medical device.

4. The delivery member of claim 1, wherein the distal hypotube further comprises a first attachment slot (310) extending from the lumen of the distal hypotube to the outer surface of the distal hypotube,
wherein a first end (408) of a loop wire extends at least partially through the first attachment slot,
optionally wherein the distal hypotube further comprises a second attachment slot (316) extending from the lumen to the outer surface,
wherein a second end (409) of the loop wire extends at least partially through the second attachment slot, and
wherein the first tube window is positioned proximal to the first attachment slot and the second attachment slot in relation to the distal end of the distal hypotube.

5. The delivery member of claim 1, wherein the length of the first tube window is from approximately 2.54 mm (0.10 inches) to 10.16 mm (0.40 inches), and the width of the first tube window is from approximately 0.51 mm (0.02 inches) to 2.54 mm (0.10 inches).

6. The delivery member of claim 1, wherein the length of the first tube window is from approximately 5.08 mm (0.2 inches) to 7.2 mm (0.30 inches), the width of the first tube window is from approximately 0.89 mm (0.035 inches) to 21.84 mm (0.86 inches).

7. The delivery member of claim 1, wherein the distal hypotube further comprises a spiral cut (307) along the longitudinal axis, and wherein the first tube window is positioned between adjacent cuts of the spiral cut.

8. The delivery member of claim 1, wherein the distal hypotube further comprises a second tube window (722), and wherein the first tube window and the second tube window are positioned equidistant from the distal end of the distal hypotube.

9. The delivery member of claim 1, wherein a distal end of the first tube window is positioned approximately 22 mm from the distal end of the distal hypotube.

10. A method of manufacturing a delivery member (10) for an implantable medical device (12), the method comprising:
passing a support tube (600) through a lumen (308) of a flexible distal hypotube (300) of the delivery member until a distal end (752) of the support tube is positioned within a length of and viewable through a tube window (720) of the flexible distal hypotube;
affixing the support tube to the delivery member; and
affixing a loop wire (400) to the flexible distal hypotube,
wherein the tube window has a length greater than a width, and wherein the length of the tube window is along a longitudinal axis passing through the lumen.

11. The method of claim 10, wherein affixing the loop wire to the flexible distal hypotube comprises:
passing a first end (408) of the loop wire through a proximal end (314) of a first attachment slot (310) of the flexible distal hypotube;
pulling the first end of the loop wire from the proximal end of the first attachment slot toward a distal end (312) of the first attachment slot, the distal end having a smaller diameter than the proximal end;
attaching the loop wire to the first attachment slot;
passing a second end (409) of the loop wire through a proximal end (324) of a second attachment slot (316) of the flexible distal hypotube;
pulling the second end of the loop wire from the proximal end of the second attachment slot toward a distal end (322) of the second attachment slot, the distal end having a smaller diameter than the proximal end; and
attaching the loop wire to the second attachment slot.

12. The method of claim 11, wherein the tube window is positioned proximal to the first attachment slot and the second attachment slot in relation to the distal end of the flexible distal hypotube.

13. The method of claim 10, further comprising forming the tube window in the flexible distal hypotube by cutting or etching the tube window between adjacent cuts of a spiral cut in the flexible distal hypotube.

14. The method of claim 10, wherein the length of the tube window is from approximately 2.54 mm (0.10 inches) to 10.16 mm (0.40 inches), and the width of the tube window is from approximately 0.51 mm (0.02 inches) to 2.54 mm (0.10 inches), or, wherein the length of the tube window is from approximately 5.08 mm (0.2 inches) to 7.2 mm (0.30 inches), the width of the tube window is from approximately 0.89 mm (0.035 inches) to 21.84 mm (0.86 inches).

15. The method of claim 10, wherein a distal end of the tube window is positioned approximately 22 mm from the distal end of the flexible distal hypotube.

## Patentansprüche

1. Abgabeelement (10), umfassend:
einen Hypotube (300), umfassend:
ein distales Ende (304), das geformt ist, um eine implantierbare medizinische Vorrichtung (12) aufzunehmen;
eine Längsachse von dem distalen Ende zu einem proximalen Ende des distalen Hypotubes; und
ein erstes Röhrenfenster (720), das sich von einem Lumen (308) des distalen Hypotubes zu einer Außenoberfläche (309) des distalen Hypotubes erstreckt, wobei das erste Röhrenfenster eine Länge aufweist, die größer als eine Breite ist, wobei die Länge entlang der Längsachse verläuft; und
eine Stützröhre (600), die innerhalb des Lumens des distalen Hypotubes positioniert ist
**dadurch gekennzeichnet, dass**
die Stützröhre durch das Lumen des distalen Hypotubes derart vorschiebbar ist, dass ein distales Ende (752) der Stützröhre innerhalb der Länge des ersten Röhrenfensters positioniert werden kann und durch dieses betrachtbar ist.

2. Abgabeelement nach Anspruch 1, wobei das distale Ende der Stützröhre derart positioniert ist, dass es innerhalb der Länge des ersten Röhrenfensters angeordnet ist und außerhalb des distalen Hypotubes sichtbar ist.

3. Abgabeelement nach Anspruch 1, ferner umfassend:
einen Schleifendraht (400), der sich durch eine Öffnung in der implantierbaren medizinischen Vorrichtung erstreckt; und
einen Zugdraht (140), der sich durch das Lumen erstreckt, der mit dem Schleifendraht in Eingriff steht und bewegbar ist, um proximal zurückgezogen zu werden, um den Schleifendraht zu lösen, um die implantierbare medizinische Vorrichtung einzusetzen.

4. Abgabeelement nach Anspruch 1, wobei der distale Hypotube ferner einen ersten Befestigungsschlitz (310) umfasst, der sich von dem Lumen des distalen Hypotubes zu der Außenoberfläche des distalen Hypotubes erstreckt,
wobei sich ein erstes Ende (408) eines Schleifendrahts mindestens teilweise durch den ersten Befestigungsschlitz erstreckt,
optional wobei der distale Hypotube ferner einen zweiten Befestigungsschlitz (316) umfasst, der sich von dem Lumen zu der Außenoberfläche erstreckt,
wobei sich ein zweites Ende (409) des Schleifendrahts mindestens teilweise durch den zweiten Befestigungsschlitz erstreckt, und
wobei das erste Röhrenfenster proximal zu dem ersten Befestigungsschlitz und dem zweiten Befestigungsschlitz in Bezug auf das distale Ende des distalen Hypotube positioniert ist.

5. Abgabeelement nach Anspruch 1, wobei die Länge des ersten Röhrenfensters von etwa 2,54 mm (0,10 Zoll) bis 10,16 mm (0,40 Zoll) beträgt und die Breite des ersten Röhrenfensters von etwa 0,51 mm (0,02 Zoll) bis 2,54 mm (0,10 Zoll) beträgt.

6. Abgabeelement nach Anspruch 1, wobei die Länge des ersten Röhrenfensters von etwa 5,08 mm (0,2 Zoll) bis 7,2 mm (0,30 Zoll) beträgt, die Breite des ersten Röhrenfensters von etwa 0,89 mm (0,035 Zoll) bis 21,84 mm (0,86 Zoll) beträgt.

7. Abgabeelement nach Anspruch 1, wobei der distale Hypotube ferner einen Spiralschnitt (307) entlang der Längsachse umfasst, und wobei das erste Röhrenfenster zwischen angrenzenden Schnitten des Spiralschnitts positioniert ist.

8. Abgabeelement nach Anspruch 1, wobei der distale Hypotube ferner ein zweites Röhrenfenster (722) umfasst, und wobei das erste Röhrenfenster und das zweite Röhrenfenster abstandsgleich von dem distalen Ende des distalen Hypotubes positioniert sind.

9. Abgabeelement nach Anspruch 1, wobei ein distales Ende des ersten Röhrenfensters etwa 22 mm von dem distalen Ende des distalen Hypotubes positioniert ist.

10. Verfahren zum Herstellen eines Abgabeelements (10) für eine implantierbare medizinische Vorrichtung (12), das Verfahren umfassend:
Führen einer Stützröhre (600) durch ein Lumen (308) eines flexiblen distalen Hypotubes (300) des Abgabeelements, bis ein distales Ende (752) der Stützröhre innerhalb einer Länge eines Röhrenfensters (720) des flexiblen distalen Hypotubes positioniert und durch dieses betrachtbar ist;
Fixieren der Stützröhre an dem Abgabeelement; und
Fixieren eines Schleifendrahts (400) an dem flexiblen distalen Hypotube,
wobei das Röhrenfenster eine Länge aufweist, die größer als eine Breite ist, und wobei die Länge des Röhrenfensters entlang einer Längsachse liegt, die durch das Lumen führt.

11. Verfahren nach Anspruch 10, wobei das Fixieren des Schleifendrahts an dem flexiblen distalen Hypotube umfasst:
Führen eines ersten Endes (408) des Schleifendrahts durch ein proximales Ende (314) eines ersten Befestigungsschlitzes (310) des flexiblen distalen Hypotubes;
Ziehen des ersten Endes des Schleifendrahts von dem proximalen Ende des ersten Befestigungsschlitzes zu einem distalen Ende (312) des ersten Befestigungsschlitzes hin, wobei das distale Ende einen geringeren Durchmesser als das proximale Ende aufweist;
Befestigen des Schleifendrahts an dem ersten Befestigungsschlitz;
Führen eines zweiten Endes (409) des Schleifendrahts durch ein proximales Ende (324) eines zweiten Befestigungsschlitzes (316) des flexiblen distalen Hypotubes;
Ziehen des zweiten Endes des Schleifendrahts von dem proximalen Ende des zweiten Befestigungsschlitzes zu einem distalen Ende (322) des zweiten Befestigungsschlitzes hin, wobei das distale Ende einen geringeren Durchmesser als das proximale Ende aufweist; und
Befestigen des Schleifendrahts an dem zweiten Befestigungsschlitz.

12. Verfahren nach Anspruch 11, wobei das Röhrenfenster in Bezug auf das distale Ende des flexiblen distalen Hypotubes proximal zu dem ersten Befestigungsschlitz und dem zweiten Befestigungsschlitz positioniert ist.

13. Verfahren nach Anspruch 10, ferner umfassend ein Ausbilden des Röhrenfensters in dem flexiblen distalen Hypotube durch ein Schneiden oder ein Ätzen des Röhrenfensters zwischen angrenzenden Schnitten eines Spiralschnitts in dem flexiblen distalen Hypotube.

14. Verfahren nach Anspruch 10, wobei die Länge des Röhrenfensters von etwa 2,54 mm (0,10 Zoll) bis 10,16 mm (0,40 Zoll) beträgt, und die Breite des Röhrenfensters von etwa 0,51 mm (0,02 Zoll) bis 2,54 mm (0,10 Zoll) beträgt, oder, wobei die Länge des Röhrenfensters von etwa 5,08 mm (0,2 Zoll) bis 7,2 mm (0,30 Zoll) beträgt, die Breite des Röhrenfensters von etwa 0,89 mm (0,035 Zoll) bis 21,84 mm (0,86 Zoll) beträgt.

15. Verfahren nach Anspruch 10, wobei ein distales Ende des Röhrenfensters etwa 22 mm von dem distalen Ende des flexiblen distalen Hypotubes positioniert ist.

## Revendications

1. Élément de pose (10) comprenant :
un hypotube distal (300) comprenant :
une extrémité distale (304) façonnée pour recevoir un dispositif médical implantable (12) ;
un axe longitudinal de l'extrémité distale à une extrémité proximale de l'hypotube distal ; et
une première fenêtre de tube (720) s'étendant depuis une lumière (308) de l'hypotube distal jusqu'à une surface externe (309) de l'hypotube distal, la première fenêtre de tube ayant une longueur supérieure à une largeur, dans lequel la longueur est le long de l'axe longitudinal ; et
un tube de support (600) positionné dans la lumière de l'hypotube distal
**caractérisé en ce que**
le tube de support peut être avancé à travers la lumière de l'hypotube distal de telle sorte qu'une extrémité distale (752) du tube de support peut être positionnée dans la longueur de la première fenêtre de tube et être visible à travers celle-ci.

2. Élément de pose selon la revendication 1, dans lequel l'extrémité distale du tube de support est positionnée de telle sorte qu'elle est placée dans la longueur de la première fenêtre de tube et est visible à l'extérieur de l'hypotube distal.

3. Élément de pose selon la revendication 1, comprenant en outre :
un fil en boucle (400) étendu à travers une ouverture dans le dispositif médical implantable ; et
un fil de traction (140) étendu à travers la lumière, en prise avec le fil en boucle et mobile pour se rétracter de manière proximale pour désolidariser le fil en boucle pour déployer le dispositif médical implantable.

4. Élément de pose selon la revendication 1, dans lequel l'hypotube distal comprend en outre une première fente d'attache (310) s'étendant de la lumière de l'hypotube distal à la surface externe de l'hypotube distal,
dans lequel une première extrémité (408) d'un fil en boucle s'étend au moins partiellement à travers la première fente d'attache,
éventuellement dans lequel l'hypotube distal comprend en outre une seconde fente d'attache (316) s'étendant de la lumière à la surface externe,
dans lequel une seconde extrémité (409) du fil en boucle s'étend au moins partiellement à travers la seconde fente d'attache, et
dans lequel la première fenêtre de tube est positionnée de manière proximale par rapport à la première fente d'attache et à la seconde fente d'attache par rapport à l'extrémité distale de l'hypotube distal.

5. Élément de pose selon la revendication 1, dans lequel la longueur de la première fenêtre de tube est d'environ 2,54 mm (0,10 pouce) à 10,16 mm (0,40 pouce), et la largeur de la première fenêtre de tube est d'environ 0,51 mm (0,02 pouce) à 2,54 mm (0,10 pouce).

6. Élément de pose selon la revendication 1, dans lequel la longueur de la première fenêtre de tube est d'environ 5,08 mm (0,2 pouce) à 7,2 mm (0,30 pouce), la largeur de la première fenêtre de tube est d'environ 0,89 mm (0,035 pouce) à 21,84 mm (0,86 pouce).

7. Élément de pose selon la revendication 1, dans lequel l'hypotube distal comprend en outre une découpe en spirale (307) le long de l'axe longitudinal, et dans lequel la première fenêtre de tube est positionnée entre des découpes adjacentes de la découpe en spirale.

8. Élément de pose selon la revendication 1, dans lequel l'hypotube distal comprend en outre une seconde fenêtre de tube (722), et dans lequel la première fenêtre de tube et la seconde fenêtre de tube sont positionnées à équidistance de l'extrémité distale de l'hypotube distal.

9. Élément de pose selon la revendication 1, dans lequel une extrémité distale de la première fenêtre de tube est positionnée à environ 22 mm de l'extrémité distale de l'hypotube distal.

10. Procédé de fabrication d'un élément de pose (10) pour un dispositif médical implantable (12), le procédé comprenant :
le passage d'un tube de support (600) à travers une lumière (308) d'un hypotube distal flexible (300) de l'élément de pose jusqu'à ce qu'une extrémité distale (752) du tube de support soit positionnée sur une longueur d'une fenêtre de tube (720) de l'hypotube distal flexible et visible à travers celle-ci ;
la fixation du tube de support à l'élément de pose ; et
la fixation d'un fil en boucle (400) à l'hypotube distal flexible,
dans lequel la fenêtre de tube a une longueur supérieure à une largeur, et dans lequel la longueur de la fenêtre de tube est le long d'un axe longitudinal passant à travers la lumière.

11. Procédé selon la revendication 10, dans lequel la fixation du fil en boucle à l'hypotube distal flexible comprend :
le passage d'une première extrémité (408) du fil en boucle à travers une extrémité proximale (314) d'une première fente d'attache (310) de l'hypotube distal flexible ;
la traction de la première extrémité du fil en boucle depuis l'extrémité proximale de la première fente d'attache vers une extrémité distale (312) de la première fente d'attache, l'extrémité distale ayant un diamètre plus petit que l'extrémité proximale ;
l'attache du fil en boucle à la première fente d'attache ;
le passage d'une seconde extrémité (409) du fil en boucle à travers une extrémité proximale (324) d'une seconde fente d'attache (316) de l'hypotube distal flexible ;
la traction de la seconde extrémité du fil en boucle depuis l'extrémité proximale de la seconde fente d'attache vers une extrémité distale (322) de la seconde fente d'attache, l'extrémité distale ayant un diamètre plus petit que l'extrémité proximale ; et
l'attache du fil en boucle à la seconde fente d'attache.

12. Procédé selon la revendication 11, dans lequel la fenêtre de tube est positionnée de manière proximale par rapport à la première fente d'attache et à la seconde fente d'attache par rapport à l'extrémité distale de l'hypotube distal flexible.

13. Procédé selon la revendication 10, comprenant en outre la formation de la fenêtre de tube dans l'hypotube distal flexible par découpe ou gravure de la fenêtre de tube entre des découpes adjacentes d'une découpe en spirale dans l'hypotube distal flexible.

14. Procédé selon la revendication 10, dans lequel la longueur de la fenêtre de tube est d'environ 2,54 mm (0,10 pouce) à 10,16 mm (0,40 pouce), et la largeur de la fenêtre de tube est d'environ 0,51 mm (0,02 pouce) à 2,54 mm (0,10 pouce), ou, dans lequel la longueur de la fenêtre de tube est d'environ 5,08 mm (0,2 pouce) à 7,2 mm (0,30 pouce), la largeur de la fenêtre de tube est d'environ 0,89 mm (0,035 pouce) à 21,84 mm (0,86 pouce).

15. Procédé selon la revendication 10, dans lequel une extrémité distale de la fenêtre de tube est positionnée à environ 22 mm de l'extrémité distale de l'hypotube distal flexible.
